(19) Europäisches Patentamt
European Patent Office
Office européen des brevets

(11) EP 4 744 657 A1

# (12) EUROPEAN PATENT APPLICATION

published in accordance with Art. 153(4) EPC

(43) Date of publication:
**20.05.2026 Bulletin 2026/21**

(21) Application number: **24843070.4**

(22) Date of filing: **12.07.2024**

(51) International Patent Classification (IPC):
***A61K 31/192*** (2006.01) ***A61K 31/57*** (2006.01)
***A61K 31/58*** (2006.01) ***A61K 31/436*** (2006.01)
***A61K 31/519*** (2006.01) ***A61K 31/573*** (2006.01)
***A61K 31/5377*** (2006.01) ***A61K 31/7056*** (2006.01)
***A61K 38/13*** (2006.01) ***A61K 45/00*** (2006.01)
***A61P 37/06*** (2006.01) ***A61P 43/00*** (2006.01)

(52) Cooperative Patent Classification (CPC):
**A61K 31/192; A61K 31/436; A61K 31/519; A61K 31/5377; A61K 31/57; A61K 31/573; A61K 31/58; A61K 31/7056; A61K 38/13; A61K 45/00; A61P 37/06; A61P 43/00**

(86) International application number:
**PCT/JP2024/025206**

(87) International publication number:
**WO 2025/018279 (23.01.2025 Gazette 2025/04)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **14.07.2023 JP 2023116182**
**14.07.2023 JP 2023116183**

(71) Applicants:
- **National University Corporation Okayama University**
**Kita-ku,**
**Okayama-shi,**
**Okayama 700-8530 (JP)**
- **Toko Pharmaceutical Industries Co., Ltd.**
**Tokyo 123-0865 (JP)**

(72) Inventor: **MAEDA, Yoshinobu**
**Okayama-shi, Okayama 700-8530 (JP)**

(74) Representative: **Plasseraud IP**
**104 Rue de Richelieu**
**CS92104**
**75080 Paris Cedex 02 (FR)**

## (54) THERAPEUTIC AGENT FOR HUMAN GRAFT VERSUS HOST DISEASE AND COMBINATION THEREOF

(57) In order to provide a novel therapeutic agent for human graft versus host disease and a combination thereof, a therapeutic agent of the present invention is a therapeutic agent for human graft versus host disease and contains tamibarotene or a pharmaceutically acceptable salt thereof as an active ingredient.

FIG. 4

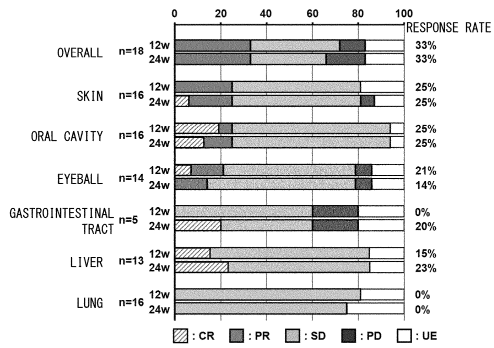




Processed by Luminess, 75001 PARIS (FR)

## Description

### Technical Field

The present invention relates to a therapeutic agent for human graft versus host disease and a combination thereof.

### Background Art

Allogeneic hematopoietic stem cell transplantation (allo-HSCT) is used for treatment of leukemia and the like. Meanwhile, graft versus host disease (GVHD) has become an issue as a complication after allo-HSCT. GVHD is roughly classified into acute GVHD and chronic GVHD based on histopathological or clinical signs (Non-Patent Literature 1).

For example, it is known that chronic GVHD occurs in approximately 40% of survivors, and the onset time is often 5 to 6 months after transplantation. A disease condition of chronic GVHD is similar to that of an autoimmune disease, and a lesion and a susceptibility to infection are seen in multiple organs such as the skin, oral cavity, eye, gastrointestinal tract, liver, joint, and lung.

Treatments for GVHD include a second-line treatment in a refractory case with respect to a first-line treatment which is initially carried out as a systemic therapy, a local therapy, a supportive therapy, and the like. A steroid therapy is carried out as a standard therapeutic method for moderate to severe GVHD. In a case where amelioration is not seen, the symptom relapses, or the like after a first-line treatment with steroid administration, a second-line treatment is carried out.

### Citation List

### [Non-patent Literature]

[Non-patent Literature 1]

Hematopoietic Cell Transplantation Guideline GVHD (Fifth edition), The Japan Society for Hematopoietic Cell Transplantation, November 2022

### Summary of Invention

### Technical Problem

However, the steroid drug causes a variety of side effects, and a patient may become, by steroid administration, steroid-dependent, intolerant to steroid, or refractory to steroid. Moreover, no standard therapeutic method has been established for the second-line treatment. Under the circumstances, it is demanded to develop a therapeutic agent with higher efficacy and safety and to establish a therapeutic method.

An object of an aspect of the present invention is to provide a novel therapeutic agent for GVHD and a combination thereof.

### Solution to Problem

In order to attain the object, the present invention includes the following aspect.

<1> A therapeutic agent for human graft versus host disease, the therapeutic agent containing tamibarotene or a pharmaceutically acceptable salt thereof as an active ingredient.

### Advantageous Effects of Invention

According to one aspect of the present invention, it is possible to provide a novel therapeutic agent for human graft versus host disease and a combination thereof.

### Brief Description of Drawings

Fig. 1 is a diagram illustrating comparisons of knee joint extension and knee joint flexion between start of administration and end of administration.

Fig. 2 is a diagram illustrating comparisons of a cutis symptom on a lower part of a back and a cutis symptom on a knee between start of administration and end of administration.

Fig. 3 is a diagram illustrating a comparison of an oral cavity symptom between start of administration and end of administration.

Fig. 4 is a diagram illustrating a response rate for each organ.

Description of Embodiments

[1. Therapeutic agent]

A therapeutic agent for human graft versus host disease (hereinafter, sometimes abbreviated as "GVHD therapeutic agent") in accordance with the present embodiment is a therapeutic agent for human graft versus host disease, the therapeutic agent containing tamibarotene or a pharmaceutically acceptable salt thereof as an active ingredient.

An example of the GVHD therapeutic agent in accordance with the present embodiment contains tamibarotene or a pharmaceutically acceptable salt thereof as an active ingredient, and is used in combination with at least one selected from a steroid drug and an immune-suppressing drug.

<Active ingredient>

Tamibarotene is a generic name for 4-[(5,6,7,8-tetrahydro-5,5,8,8-tetramethyl-2-naphthalenyl)carbamoyl]benzoic acid. An alternative name is AM80, and a drug product is commercially available under the name of Amnolake (registered trademark). A structural formula for tamibarotene is indicated below.

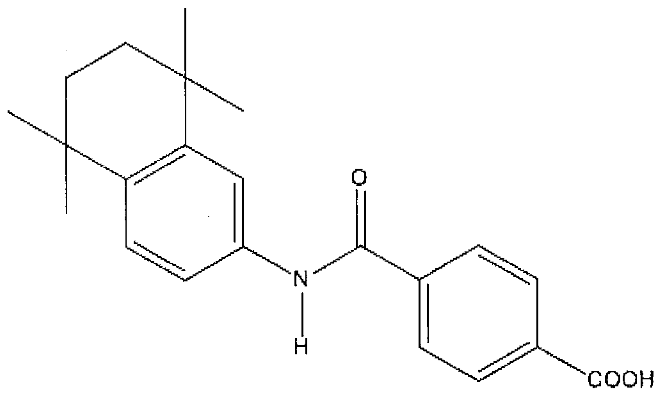

For example, tamibarotene can be produced by the methods disclosed in Japanese Patent No. 3001632, Japanese Patent Application Publication, Tokukaisho, No. 61-76440, International Publication No. 2002/018322, and the like.

<Pharmaceutically acceptable salt>

Examples of a pharmaceutically acceptable salt of tamibarotene include non-toxic, pharmaceutically acceptable, conventional salts. Specific examples include salts with an inorganic or organic base, such as alkali metal salts (such as sodium salt, potassium salt), alkaline earth metal salts (e.g., calcium salt, magnesium salt, and the like), ammonium salts, and amine salts (e.g., triethylamine salt, N-benzyl-N-methylamine salt, and the like).

Tamibarotene can also form a solvate, which is also included in the scope of tamibarotene. Preferable solvates include a hydrate and an ethanolate.

<Graft versus host disease (GVHD)>

GVHD is a rejection reaction in which, at the time of an organ transplant, lymphocytes contained in a graft and blood of a donor (organ donor) attack an organ of a recipient (who receives an organ donation). Examples of the organ include bone marrow, blood, and the like. GVHD is roughly classified into acute GVHD, which occurs in an early stage after transplantation, and chronic GVHD, which occurs in an autoimmune-like symptom in a late stage after transplantation. For acute GVHD, it has been clarified that donor T cells are differentiated and activated into Th1 cells by antigen presentation

cells of a recipient, and tissue is impaired by inflammatory cytokines and cytotoxic cells (such as T cells, macrophages). Meanwhile, for chronic GVHD, it is assumed that, in a stage in which immunity is reconstituted in a body of a recipient by donor cells, autoantigen reactive T cells appear, and a symptom which is similar to an autoimmune disease is shown, and chronic GVHD differs from acute GVHD in onset time and disease condition.

Acute GVHD and chronic GVHD are classified according to histopathological or clinical signs.

Acute GVHD is further classified into classical acute GVHD, which occurs within 100 days after transplantation, and atypical acute GVHD, which occurs on or after 100 days. Classical acute GVHD is a group that presents with typical clinical symptoms such as maculopapular rash, nausea, vomiting, emaciation, watery diarrhea, ileus, and cholestatic hepatitis. Atypical acute GVHD includes persistent acute GVHD, in which clinical condition of classical acute GVHD persists for 100 or more days, recurrent acute GVHD, in which acute GVHD relieved once relapses on or after 100 days, and late-onset acute GVHD, which occurs de novo on or after 100 days.

Meanwhile, diagnosis of chronic GVHD is made regardless of the onset time. Chronic GVHD is classified into conventional classical chronic GVHD and overlap syndrome GVHD, in which a patient once diagnosed with chronic GVHD (regardless of whether or not amelioration thereof is seen) has one or more acute GVHD symptoms. Diagnosis of overlap syndrome GVHD is made in a case where there is coexisting acute GVHD at the time of diagnosis of chronic GVHD, a case where acute GVHD appears after diagnosis of chronic GVHD, or a case where, even if a chronic GVHD symptom has been ameliorated, acute GVHD relapses in a patient who has been diagnosed once.

The GVHD therapeutic agent in accordance with the present embodiment can be suitably used for treatment of chronic GVHD in terms of being able to suppress production of Th1 and Th17, which are cytokines.

Moreover, the GVHD therapeutic agent in accordance with the present embodiment can be suitably used to ameliorate a lesion derived from GVHD or suppress progression of the lesion in at least one selected from the group consisting of skin, an oral cavity, eyeballs, fasciae, joints, and lungs.

The term "treatment" as used herein refers to relieving or eliminating an illness, disease, or disorder that is already affecting a patient, or a symptom associated therewith. Further, the term "treatment" as used herein also includes preventing contracting an illness, disease, or disorder.

<Other optional ingredients>

The GVHD therapeutic agent in accordance with the present embodiment may contain one or more therapeutically active substances having therapeutic action on another illness, disease, and condition, in addition to the active ingredient described above, provided that such an active substance is not likely to inhibit activity of the active ingredient and is not harmful to a subject to receive administration (hereinafter also referred to as a patient).

The GVHD therapeutic agent in accordance with the present embodiment may also contain a pharmaceutically acceptable carrier that is not harmful to a subject to receive administration. The carrier which can be used may be either solid, semi-solid, or liquid. Examples thereof include, but not limited to, any one selected from water, an electrolyte solution, a sugar solution, and the like. Furthermore, the therapeutic agent may contain an adjuvant. Examples of the adjuvant include a lubricant, a stabilizer, an antiseptic, an emulsifier, a thickener (viscosity modifier), a colorant, a flavoring agent, an excipient, a preservative, a buffer, a taste-masking agent, a suspending agent, an emulsifier, a solubilizing agent, a lubricant, and the like.

<Dosage form>

Examples of the dosage form include a tablet, a capsule, a pill, a granule, a powder, a syrup, a suppository, eye drops, an aerosol, a troche, a pellet, an emulsion, a suspension, and other known forms. Among these, the dosage form is preferably, for example, any one of a tablet, a capsule, a pill, a granule, a powder, a solution, a syrup, and a jelly, more preferably any one of a tablet, a capsule, and a granule, further preferably a tablet, as an oral administration formulation. As discussed later, for example, the dosage form may be a parenteral administration formulation such as an injectable formulation, a suppository, and a transdermal absorption formulation such as a patch.

<Method for producing therapeutic agent>

The GVHD therapeutic agent in accordance with the present embodiment can be produced using a known production method. For example, an active ingredient and any other ingredient are separately produced for each ingredient, and then the ingredients are mixed to have respective intended contents, and thus the therapeutic agent is produced.

<Subject to receive administration of therapeutic agent>

A subject to receive administration of the GVHD therapeutic agent in accordance with the present embodiment is a human subject who is suffering from GVHD or is likely to suffer from GVHD.

<Administration route/administration method>

An administration method (administration route) of the GVHD therapeutic agent in accordance with the present embodiment can be decided, as appropriate, according to an age, a condition, a treatment period, and the like of a subject to receive administration. Specifically, the administration method may be either oral administration or parenteral administration, and it is preferable to use oral administration (in Examples, oral administration is employed). Examples of the parenteral administration include methods such as injection administration, administration as a suppository, and administration as a transdermal absorption formulation such as a patch. Examples of a type of injection administration include intramuscular injection, intraperitoneal injection, subcutaneous injection, intravenous injection, and local injection. The GVHD therapeutic agent in accordance with the present embodiment can be administered via various routes, such as transdermal, nasal, vaginal, and rectal routes.

A dose, an administration time, an administration period, and a timing of administration of the therapeutic agent in accordance with the present embodiment will be described in detail in the section [3. Treatment method for GVHD] later.

<Combination use with at least one selected from steroid drug and immune-suppressing drug>

The GVHD therapeutic agent in accordance with the present embodiment may be used in combination with at least one selected from a steroid drug and an immune-suppressing drug. Thus, the GVHD therapeutic agent in accordance with an embodiment is used in combination with at least one selected from a steroid drug and an immune-suppressing drug.

The term "combination use" includes use at any timing prior to, concurrently with, or after use of at least one selected from a steroid drug and an immune-suppressing drug. The term "combination use" also includes a combination drug containing a steroid drug. By use in combination with at least one selected from a steroid drug and an immune-suppressing drug, a steroid dose or an immune-suppressing drug dose can be reduced as compared with that in administration of a steroid or an immune-suppressing drug alone.

For example, it is possible to reduce a daily dose of the steroid drug used in combination with the GVHD therapeutic agent in accordance with the present embodiment, as compared with a daily dose of the steroid drug needed when administered alone. Specifically, a daily dose of the steroid drug used in combination with the GVHD therapeutic agent in accordance with the present embodiment can be preferably 90% or less, more preferably 50% or less, and further preferably 10% or less, as compared with a daily dose of the steroid drug needed when administered alone. That is, the GVHD therapeutic agent in accordance with the present embodiment can reduce a daily dose of the steroid drug used in combination by preferably 10% or more, more preferably 50% or more, and further preferably 90% or more, as compared with a daily dose of the steroid drug needed when used alone for treatment.

Moreover, at or after 24 weeks from the start of administration of the GVHD therapeutic agent in accordance with the present embodiment, a daily dose of the steroid drug used in combination with the therapeutic agent can be preferably 90% or less, more preferably 50% or less, and further preferably 10% or less, as compared with a daily dose of the steroid drug used in combination at the start of administration of the therapeutic agent. That is, at or after 24 weeks from the start of administration of the GVHD therapeutic agent in accordance with the present embodiment, the therapeutic agent can reduce a daily dose of the steroid drug used in combination by preferably 10% or more, more preferably 50% or more, and further preferably 90% or more, as compared with a daily dose of the steroid drug used in combination at the start of administration of the therapeutic agent.

The daily dose of the steroid drug used in combination with the GVHD therapeutic agent in accordance with the present embodiment may be reduced stepwise in accordance with a disease condition of a subject and the like.

An example of the GVHD therapeutic agent in accordance with the present embodiment is to be administered to any one of the following subjects 1) through 3), for whom a dose reduction of the steroid drug is difficult. By administering the GVHD therapeutic agent in accordance with the present embodiment, a dose of the steroid drug administered to the subject can be reduced.

1) A subject for whom a daily dose of the steroid drug is difficult to reduce from 10 mg/kg in terms of prednisolone;
2) a subject for whom exacerbation is seen even when a daily dose of the steroid drug is 1 mg/kg in terms of prednisolone and to whom the steroid drug has been administered daily for 2 weeks; and
3) a subject whose symptom is not ameliorated even when a daily dose of the steroid drug is 0.5 mg/kg in terms of prednisolone and the steroid drug has been administered daily for 4 to 8 weeks.

Herein, the "dose in terms of prednisolone" means a dose of the steroid drug obtained when the steroid dose is converted to a dose of prednisolone, which has a drug efficacy equivalent to that of the steroid drug.

Examples of the steroid drug include prednisolone, methylprednisolone, clobetasol propionate, diflorasone acetate, betamethasone dipropionate, diflubrednate, diflucortolone valerate, fluocinonide, amcinonide, hydrocortisone butyrate propionate, betamethasone acetate propionate, mometasone furoate, betamethasone valerate-gentamicin sulfate, betamethasone valerate, dexamethasone propionate, beclomethasone propionate, dexamethasone valerate, fluocinolone acetonide, debrodone propionate, triamcinolone acetonide, hydrocortisone butyrate, clobetasone butyrate, alclometasone propionate, prednisolone valerate acetate, dexamethasone, and the like.

Examples of the immune-suppressing drug include cyclosporine, tacrolimus, mycophenolate mofetil, sirolimus, pentostatin, methotrexate, and the like.

For example, the GVHD therapeutic agent in accordance with the present embodiment may be used in combination with a pharmaceutical agent having an additional GVHD therapeutic effect or a pharmaceutical agent having an alternative effect, unless such a pharmaceutical agent impairs the GVHD therapeutic effect of the GVHD therapeutic agent.

In still another embodiment, the therapeutic agent has therapeutic effects not only on GVHD but also on complications associated with GVHD. Examples of the complications include bronchitis, pneumonia, opportunistic infection, thrombosis, osteoporosis, and the like.

The GVHD therapeutic agent in accordance with yet another embodiment of the present invention can be in a form of a prophylactic agent which is administered prior to the onset of GVHD to prevent the onset of GVHD described above. Such a prophylactic agent has a clinically important significance of preventing the symptom of GVHD from becoming severe.

For chronic GVHD, treatment with a steroid drug is standard as a first-line treatment. It is considered to be reasonable to reduce a dose of the steroid drug as a second-line treatment. The GVHD therapeutic agent in accordance with the present embodiment is expected to play a role as a second-line therapeutic agent for chronic GVHD.

The GVHD therapeutic agent in accordance with the present embodiment can significantly reduce a dose of the steroid drug used in combination with the GVHD therapeutic agent. Therefore, there is also a possibility that a low dose steroid treatment for a longer term can be applied by use in combination with the GVHD therapeutic agent in accordance with the present embodiment. Moreover, it is possible to reduce the occurrence of various adverse events that occur in association with the use of a steroid drug.

(2. Combination of therapeutic agent)

The present invention also provides a combination of a therapeutic agent for GVHD, the combination including at least one selected from a steroid drug and an immune-suppressing drug, and the GVHD therapeutic agent in accordance with the present invention (hereinafter sometimes abbreviated simply as "combination" below).

Examples of the steroid drug and the immune-suppressing drug are as exemplified above.

In a combination in accordance with one embodiment, a therapeutic agent for GVHD and at least one selected from a steroid drug and an immune-suppressing drug are a combination of separate pharmaceutical agents for using these pharmaceutical agents as a combination. For example, the therapeutic agent for GVHD and the immune-suppressing drug may be in the form of separate compositions. For other ingredients contained in the composition and dosage forms, those described in [1. Therapeutic agent] above can be applied.

The combination can be suitably used for a therapeutic method for GVHD in accordance with the present embodiment. Specific examples of the combination include the following combination: a therapeutic agent for GVHD, a steroid drug, and an immune-suppressing drug are prepared as pharmaceutical agents for treating GVHD to which drug labels are attached, respectively, and these pharmaceutical agents are used in combination. The labels of the pharmaceutical agents may indicate that the pharmaceutical agents may be used in combination with each other.

In a case where the therapeutic agent for GVHD and at least one selected from a steroid drug and an immune-suppressing drug are used in the form of separate pharmaceutical agents, the compositions may contain the same ingredient as another ingredient, or may contain different ingredients.

The combination of the pharmaceutical agent for treating GVHD in accordance with the present embodiment can be used to carry out administration of the therapeutic agent for GVHD and administration of at least one selected from a steroid drug and an immune-suppressing drug in appropriate doses at appropriate times, respectively, in accordance with a change in disease condition of a subject.

The combination of the therapeutic agent for GVHD in accordance with the present embodiment can be used for both long-term administration and short-term administration. Therefore, the combination can be suitably used for treatment of GVHD disease.

[3. Treatment method for GVHD]

A method for treating GVHD using the GVHD therapeutic agent in accordance with the present embodiment is also within the scope of the present embodiment. The following description will discuss details of a method for treating GVHD in accordance with the present embodiment.

The method for treating GVHD in accordance with an embodiment includes a step of administering the GVHD therapeutic agent in accordance with the present embodiment to a human subject (GVHD therapeutic agent administration step). The treatment of GVHD means ameliorating a symptom caused by GVHD to a better condition or a normal condition, and is a concept including prevention of the onset of GVHD.

Another method for treating GVHD is a combination therapy using a GVHD therapeutic agent and at least one selected from a steroid drug and an immune-suppressing drug. In one example, the treatment method for GVHD is a combination therapy with a steroid drug. That is, the method for treating GVHD includes at least one of a step of administering a steroid drug to a human subject (steroid drug administration step) and a step of administering an immune-suppressing drug to a human subject (immune-suppressing drug administration step), in addition to the GVHD therapeutic agent administration step.

The therapeutic agent to be administered also contains, as an active ingredient, tamibarotene or a pharmaceutically acceptable salt thereof. The GVHD therapeutic agent in accordance with the present embodiment also encompasses those described in the section [1. Therapeutic agent] above. Moreover, a subject to receive the therapeutic method in accordance with the present embodiment is as described in the section of <Subject to receive administration of therapeutic agent> in [1. Therapeutic agent] above. An example of the therapeutic method is a therapeutic method which is applied to a human subject.

As the steroid drug and the immune-suppressing drug, those described in the section [1. Therapeutic agent] are applicable.

The following description will discuss details of each step.

(GVHD therapeutic agent administration step)

The GVHD therapeutic agent administration step is a step of administering the GVHD therapeutic agent in accordance with the present embodiment to a subject.

(GVHD therapeutic agent dose)

The dose of the therapeutic agent varies depending on a type of GVHD, a severity, results of various tests, a type of active ingredient of the therapeutic agent, and the like in a patient who undergoes administration of the therapeutic agent. Moreover, the dose of the therapeutic agent varies depending on an age of a patient to be treated, the number of times a treatment with the therapeutic method in accordance with the present embodiment is carried out, various test results, and the like. For example, the GVHD therapeutic agent in accordance with the present embodiment is administered at a dose lower than that used as an immune-suppressing drug in a live organ transplant or a treatment of an immune system disease or the like, from the viewpoint of a content of the active ingredient contained in the therapeutic agent. For example, in a case where the subject to receive administration of the GVHD therapeutic agent is a human, a daily dose of the active ingredient is not particularly limited and is preferably 0.5 to 10.0 mg, more preferably 0.5 to 8.0 mg, further preferably 0.5 to 6.0 mg, and furthermore preferably 0.5 to 4.0 mg. Particularly preferably, the dose is 2.0 to 4.0 mg. Hereinafter, unless otherwise specified, the description pertaining to a therapeutic agent dose is applied to a case where the subject is a human, and the dose is expressed as an amount of the active ingredient.

In an embodiment, a plasma concentration of the active ingredient in the GVHD therapeutic agent is maintained at preferably 1 ng/mL to 200 ng/mL, more preferably 2 ng/mL to 200 ng/mL in a subject receiving a treatment during a dosing period.

For example, a lowest plasma concentration of the active ingredient is maintained at 1 ng/mL or more, 2 ng/mL or more, 5 ng/mL or more, or 8 ng/mL or more during the dosing period. A highest plasma concentration of the active ingredient is maintained at 200 ng/mL or less, 180 ng/mL or less, 150 ng/mL or less, or 100 ng/mL or less during the dosing period.

During a dose reduction period, the plasma concentration of the active ingredient may be maintained at 1 ng/mL or more, or at 2 ng/mL or more, when administered to a subject receiving a treatment. During a drug holiday period, the plasma concentration of the active ingredient in a subject receiving a treatment may be 0 ng/mL. The dosing period, dose reduction period, and drug holiday period will be described later.

In a case of oral administration, the number of daily administrations is not particularly limited and is preferably 1 to 4 times, more preferably 1 to 3 times, further preferably 1 to 2 times. A time of administration within a day is not particularly limited, and the time can be, for example, any one of before a meal, after a meal, and between meals.

(Time of administration of GVHD therapeutic agent and administration period)

The administration time and administration period of the GVHD therapeutic agent in accordance with the present embodiment varies depending on a type of an illness, disease, or condition, a severity, results of various tests, a type of active ingredient of the therapeutic agent, and the like in a patient who undergoes administration of the therapeutic agent. Moreover, the dose of the therapeutic agent varies depending on an age of a patient to be treated, the number of times a treatment with the therapeutic method in accordance with the present embodiment is carried out, various test results, and the like. For example, in a case where the subject to receive administration of the therapeutic agent is a human, the dosing interval is not particularly limited and is preferably 3 to 180 days, more preferably 60 to 180 days, and further preferably 120 to 180 days.

The GVHD therapeutic agent in accordance with the present embodiment may be administered to a subject with a dosing period, a drug holiday period, or a dose reduction period provided in the administration period. The dosing period stands for a period during which a drug is actually administered. The drug holiday period stands for a period during which no drug administration is carried out for 1 day or more. The dose reduction period stands for a period during which a dose is reduced from that used at the start of treatment. The drug holiday period or the dose reduction period can be selected appropriately in accordance with a disease condition of a subject and the like. The drug holiday period or dose reduction period is preferably 14 to 56 days, more preferably 14 to 28 days, and further preferably 14 to 21 days. The drug holiday period and the dosing period may be repeated. The period for administration of the dose used at the start of treatment and the dose reduction period may be repeated.

(GVHD therapeutic agent administration period)

The administration period is not particularly limited, and administration may be carried out continuously or intermittently.

In a case where the method in accordance with the present embodiment is a method for treating GVHD in a human subject, the GVHD therapeutic agent administration period in the administration step is preferably 4 months to 6 months, more preferably 2 months to 6 months, and further preferably 1 month to 6 months.

The administration in the above period may be either continuous (i.e., administered daily during the period) or intermittent (i.e., a day without administration may be included in the period). Specifically, the description of [1. Therapeutic agent] (the time and period of administration of the therapeutic agent) is applied. Specific examples of the intermittent administration include a form of administration at regular intervals of predetermined days (1 day, 2 days, 3 days), and a form of administration with one or more predetermined holiday periods each consisting of multiple days.

(Steroid drug administration step and immune-suppressing drug administration step)

The steroid drug administration step is a step of administering a steroid drug to a human subject. The immune-suppressing drug administration step is a step of administering an immune-suppressing drug to a human subject.

(Dose of steroid drug)

The dose of the steroid drug varies depending on a type of GVHD, a severity, results of various tests, a type of active ingredient of the therapeutic agent, and the like in a patient who undergoes administration of the GVHD therapeutic agent. Moreover, the dose of the therapeutic agent varies depending on an age of a patient to be treated, the number of times a treatment with the therapeutic method in accordance with the present embodiment is carried out, various test results, and the like. For example, the steroid drug is administered at a dose lower than that used as an immune-suppressing drug in a live organ transplant or a treatment of an immune system disease or the like, from the viewpoint of a content of the active ingredient contained in the therapeutic agent. For example, in a case where the subject to receive administration of the GVHD therapeutic agent is a human, a daily dose in terms of prednisolone is not particularly limited and is preferably 1.0 to 60.0 mg, more preferably 1.0 to 40.0 mg, further preferably 1.0 to 20.0 mg.

(Dose of immune-suppressing drug)

The dose of the immune-suppressing drug varies depending on a type of GVHD and the like in a patient who undergoes administration of the GVHD therapeutic agent, as with the steroid drug.

(Timing of combination administration of GVHD therapeutic agent and at least one selected from steroid drug and immune-suppressing drug)

The GVHD therapeutic agent and at least one selected from a steroid drug and an immune-suppressing drug may be administered simultaneously or may be administered separately.

In a case where the GVHD therapeutic agent and the steroid drug are contained in separate pharmaceutical agents, the GVHD therapeutic agent and the steroid drug may be administered simultaneously, or the steroid drug may be administered before or after administration of the GVHD therapeutic agent. For example, in a case where the GVHD therapeutic agent is administered orally, the GVHD therapeutic agent may be administered, for example, within 0 to 2 hours of administration of the steroid drug, 0 to 2 hours before or 0 to 2 hours after administration of the GVHD therapeutic agent.

In the GVHD therapeutic agent administration step and at least one step selected from the steroid drug administration step and the immune-suppressing drug administration step, the GVHD therapeutic agent and at least one selected from the steroid drug and the immune-suppressing drug may be administered simultaneously or as a mixture. Alternatively, the GVHD therapeutic agent and at least one selected from the steroid drug and the immune-suppressing drug may be administered at different times.

A steroid drug or an immune-suppressing drug that is administered during a certain treatment period may be only one type or a plurality of types.

Compositional ingredients to be administered may be altered in a manner in which, for example, a specific drug is administered for a certain period of administration, and then another steroid drug is administered for an additional certain period. The composition of the administered active ingredients during a certain period may be altered in an intended cycle and order.

The therapeutic method in accordance with the present embodiment may further include a step of measuring a marker pertaining to onset of thrombosis, in addition to the GVHD therapeutic agent administration step.

(Step of measuring marker pertaining to onset of thrombosis)

A step of measuring a factor involved in onset of thrombosis is a step of measuring a marker (such as a D-dimer) pertaining to onset of thrombosis.

It is known that the incidence of thrombosis is high in patients with GVHD. By measuring a marker (such as a D-dimer) pertaining to onset of thrombosis, it is possible to consider a change in the dose of the therapeutic agent or a combination use with an anticoagulant. Measurement of a marker pertaining to onset of thrombosis can be carried out using a known measurement method. For example, the measurement can be carried out by quantifying D-dimers in blood.

The therapeutic method in accordance with the present embodiment may include a step of administering an anticoagulant if needed, based on a measurement result obtained in the step of measuring a marker described above.

(Dosage schedule)

The therapeutic method in accordance with the present embodiment may be carried out in accordance with a dosage regimen of any of (1) through (5) below or a combination of (1) through (5) below.

(1) The therapeutic agent is orally administered daily at 4 mg per day for 24 consecutive weeks.
(2) In a first administration period, the therapeutic agent is orally administered daily at 4 mg per day for 4 consecutive weeks, and in a second administration period, the therapeutic agent is orally administered daily at 6 to 8 mg per day for consecutive days.
(3) In a first administration period, the therapeutic agent is orally administered daily at 4 mg per day for 4 consecutive weeks, and in a second administration period, the therapeutic agent is orally administered daily at 2 mg or less per day for consecutive days.
(4) In a first administration period, the therapeutic agent is orally administered at 4 mg per day for 4 consecutive weeks, and in a second administration period, the therapeutic agent is orally administered daily at 2 mg or less per day for consecutive days. A drug holiday period for 2 weeks is provided between the first administration period and the second administration period. The second administration period and the drug holiday period for 2 weeks are repeated in accordance with a grade of an adverse event that has occurred during the second administration period.
(5) In a first administration period, the therapeutic agent is orally administered at 4 mg per day for 4 consecutive weeks, and in a second administration period, the therapeutic agent is orally administered daily at 2 mg or less per day for consecutive days. A drug holiday period for 2 weeks is provided between the first administration period and the second administration period. The second administration period and the drug holiday period for 2 weeks are repeated in accordance with a grade of an adverse event that has occurred during the second administration period. In a third

administration period, the therapeutic agent is orally administered on alternate days at 2 mg or less per day.

The term "adverse event" refers to any unfavourable or unintended sign (including an abnormal value in clinical test), symptom, or disease that occurs when tamibarotene is administered, regardless of the presence or absence of a causal relationship with tamibarotene. Note that a finding that was present before administration of tamibarotene is not considered an adverse event if the symptom/finding has not worsened after the administration. An exacerbation of a primary disease during a period of administration of tamibarotene is not considered an adverse event if the exacerbation is within a range of expected variation and it is medically acceptable to deal with the exacerbation by a steroid dose increase within 1 mg/kg.

Evaluation of severity of an adverse event can be performed by grading in accordance with the common terminology criteria for adverse events (CTCAE).

[4. Other aspects]

In an embodiment, the present invention encompasses a method for reducing the expression of a condition or complication associated with GVHD. Examples of the condition or complication associated with GVHD include bronchitis, pneumonia, opportunistic infection, thrombosis, osteoporosis, and the like.

Use of at least one compound selected from tamibarotene or a pharmaceutically acceptable salt thereof in production of a therapeutic agent is also encompassed in the scope of the present embodiment.

[5. Examples of specific aspects of present invention]

In order to attain the object, the present invention encompasses any one of the following aspects.

<1-1> A therapeutic agent for human graft versus host disease, the therapeutic agent containing tamibarotene or a pharmaceutically acceptable salt thereof as an active ingredient.

<1-2> The therapeutic agent described in <1-1>, in which: the human graft versus host disease is chronic graft versus host disease.

<1-3> The therapeutic agent described in <1-1> or <1-2>, which suppresses progression of or ameliorates a lesion derived from the human graft versus host disease in at least one selected from the group consisting of skin, an oral cavity, eyeballs, fasciae, joints, and lungs.

<1-4> The therapeutic agent described in <1-1> through <1-3>, in which: a plasma concentration of the active ingredient is maintained at 1 ng/mL to 200 ng/mL during a dosing period in a subject receiving a treatment.

<1-5> The therapeutic agent described in <1-1> through <1-4>, which is administered to a subject with a drug holiday period or a dose reduction period.

<1-6> The therapeutic agent described in <1-5>, in which: during the dose reduction period, a plasma concentration of the active ingredient is maintained at 1 ng/mL or more in the subject to which the therapeutic agent has been administered.

<1-7> The therapeutic agent described in <1-1> through <1-6>, which is administered to any of:

1) a subject for whom a daily dose of the steroid drug is difficult to reduce from 10 mg/kg in terms of prednisolone;
2) a subject for whom exacerbation is seen even when a daily dose of the steroid drug is 1 mg/kg in terms of prednisolone and to whom the steroid drug has been administered daily for 2 weeks; and
3) a subject whose symptom is not ameliorated even when a daily dose of the steroid drug is 0.5 mg/kg in terms of prednisolone and the steroid drug has been administered daily for 4 to 8 weeks.

<2-1> A therapeutic agent for human graft versus host disease, the therapeutic agent containing tamibarotene or a pharmaceutically acceptable salt thereof as an active ingredient, and being used in combination with at least one selected from a steroid drug and an immune-suppressing drug.

<2-2> The therapeutic agent described in <2-1>, in which: the human graft versus host disease is chronic graft versus host disease.

<2-3> The therapeutic agent described in <2-1> or <2-2>, which suppresses progression of or ameliorates a lesion derived from the human graft versus host disease in at least one selected from the group consisting of skin, an oral cavity, eyeballs, fasciae, joints, and lungs.

<2-4> The therapeutic agent described in <2-1> through <2-3>, in which: the steroid drug is selected from the group consisting of prednisolone, methylprednisolone, clobetasol propionate, diflorasone acetate, betamethasone dipropionate, diflubrednate, diflucortolone valerate, fluocinonide, amcinonide, hydrocortisone butyrate propionate, betamethasone acetate propionate, mometasone furoate, betamethasone valerate-gentamicin sulfate, betamethasone

valerate, dexamethasone propionate, beclomethasone propionate, dexamethasone valerate, fluocinolone acetonide, debrodone propionate, triamcinolone acetonide, hydrocortisone butyrate, clobetasone butyrate, alclometasone propionate, prednisolone valerate acetate, and dexamethasone; and
the immune-suppressing drug is selected from the group consisting of cyclosporine, tacrolimus, mycophenolate mofetil, sirolimus, pentostatin, and methotrexate.
<2-5> The therapeutic agent described in <2-1> through <2-4>, in which: a daily dose of the steroid drug used in combination with the therapeutic agent is reduced as compared with a daily dose of the steroid drug needed when used alone for treatment.
<2-6> The therapeutic agent described in <2-1> through <2-5>, in which: a daily dose of the steroid drug used in combination with the therapeutic agent is reduced by 10% or more as compared with a daily dose of the steroid drug needed when used alone for treatment.
<2-7> The therapeutic agent described in <2-1> through <2-6>, in which: at or after 24 weeks from start of administration of the therapeutic agent, a daily dose of the steroid drug used in combination with the therapeutic agent is reduced by 10% or more as compared with a daily dose of the steroid drug used in combination with the therapeutic agent at the start of administration of the therapeutic agent.
<2-8> The therapeutic agent described in <2-1> through <2-7>, in which: a daily dose of the steroid drug used in combination with the therapeutic agent is reduced stepwise.
<2-9> The therapeutic agent described in <2-1> through <2-8>, which is administered to any of:

1) a subject for whom a daily dose of the steroid drug is difficult to reduce from 10 mg/kg in terms of prednisolone;
2) a subject for whom exacerbation is seen even when a daily dose of the steroid drug is 1 mg/kg in terms of prednisolone and to whom the steroid drug has been administered daily for 2 weeks; and
3) a subject whose symptom is not ameliorated even when a daily dose of the steroid drug is 0.5 mg/kg in terms of prednisolone and the steroid drug has been administered daily for 4 to 8 weeks.

<2-10> The therapeutic agent described in <2-1> through <2-9>, which is administered to a subject with a drug holiday period or a dose reduction period.
<2-11> A combination of a therapeutic agent for human graft versus host disease, the combination including: at least one selected from a steroid drug and an immune-suppressing drug; and the therapeutic agent described in <2-1> through <2-10>.
<3-1> A therapeutic agent for human graft versus host disease, the therapeutic agent containing tamibarotene or a pharmaceutically acceptable salt thereof as an active ingredient.
<3-2> The therapeutic agent described in <3-1> which is used in combination with at least one selected from a steroid drug and an immune-suppressing drug.
<3-3> The therapeutic agent described in <3-1> or <3-2>, in which: the human graft versus host disease is chronic graft versus host disease.
<3-4> The therapeutic agent described in any one of <3-1> through <3-3>, which suppresses progression of or ameliorates a lesion derived from the human graft versus host disease in at least one selected from the group consisting of skin, an oral cavity, eyeballs, fasciae, joints, and lungs.
<3-5> The therapeutic agent described in any one of <3-1> through <3-4>, in which: a plasma concentration of the active ingredient is maintained at 1 ng/mL to 200 ng/mL during a dosing period in a subject receiving a treatment.
<3-6> The therapeutic agent described in any one of <3-1> through <3-5>, which is administered to a subject with a drug holiday period or a dose reduction period.
<3-7> The therapeutic agent described in <3-6>, in which: during the dose reduction period, a plasma concentration of the active ingredient is maintained at 1 ng/mL or more in the subject to which the therapeutic agent has been administered.
<3-8> The therapeutic agent described in any one of <3-2> through <3-7>, in which: the steroid drug is selected from the group consisting of prednisolone, methylprednisolone, clobetasol propionate, diflorasone acetate, betamethasone dipropionate, diflubrednate, diflucortolone valerate, fluocinonide, amcinonide, hydrocortisone butyrate propionate, betamethasone acetate propionate, mometasone furoate, betamethasone valerate-gentamicin sulfate, betamethasone valerate, dexamethasone propionate, beclomethasone propionate, dexamethasone valerate, fluocinolone acetonide, debrodone propionate, triamcinolone acetonide, hydrocortisone butyrate, clobetasone butyrate, alclometasone propionate, prednisolone valerate acetate, and dexamethasone; and the immune-suppressing drug is selected from the group consisting of cyclosporine, tacrolimus, mycophenolate mofetil, sirolimus, pentostatin, and methotrexate.
<3-9> The therapeutic agent described in any one of <3-2> through <3-8>, in which: a daily dose of the steroid drug used in combination with the therapeutic agent is reduced as compared with a daily dose of the steroid drug needed when used alone for treatment.

<3-10> The therapeutic agent described in any one of <3-2> through <3-9>, in which: a daily dose of the steroid drug used in combination with the therapeutic agent is reduced by 10% or more as compared with a daily dose of the steroid drug needed when used alone for treatment.

<3-11> The therapeutic agent described in any one of <3-2> through <3-10>, in which: at or after 24 weeks from start of administration of the therapeutic agent, a daily dose of the steroid drug used in combination with the therapeutic agent is reduced by 10% or more as compared with a daily dose of the steroid drug used in combination with the therapeutic agent at the start of administration of the therapeutic agent.

<3-12> The therapeutic agent described in any one of <3-2> through <3-11>, in which: a daily dose of the steroid drug used in combination with the therapeutic agent is reduced stepwise.

<3-13> The therapeutic agent described in any one of <3-1> through <3-12>, which is administered to any of:

1) a subject for whom a daily dose of the steroid drug is difficult to reduce from 10 mg/kg in terms of prednisolone;
2) a subject for whom exacerbation is seen even when a daily dose of the steroid drug is 1 mg/kg in terms of prednisolone and to whom the steroid drug has been administered daily for 2 weeks; and
3) a subject whose symptom is not ameliorated even when a daily dose of the steroid drug is 0.5 mg/kg in terms of prednisolone and the steroid drug has been administered daily for 4 to 8 weeks.

<3-14> A combination of a therapeutic agent for human graft versus host disease, the combination including: at least one selected from a steroid drug and an immune-suppressing drug; and
the therapeutic agent described in any one of <3-2> through <3-13>.

The following description will discuss an embodiment of the present invention in further detail with reference to Examples. The present invention is of course not limited to Examples below, and various aspects may be employed for details. Further, the present invention is not limited to the embodiment, but can be altered by a skilled person in the art within the scope of the claims. The present invention also encompasses in its technical scope any embodiment based on an appropriate combination of the technical means disclosed. All literatures described in this specification are incorporated herein as reference literatures.

Examples

[Example 1: Study of efficacy and safety of tamibarotene for chronic GVHD]

Tamibarotene was administered to a patient with chronic graft versus host disease (chronic GVHD) after allogeneic hematopoietic stem cell transplantation, and efficacy thereof was studied.

<Selection criteria for subjects>

Main selection criteria for subjects are as follows:

(A) An age is 18 years or older and younger than 85 years
(B) A Karnofsky performance status (KPS) is 60 or more
(C) A steroid treatment-refractory or steroid treatment-dependent patient who has started treatment with 0.5 mg/kg or more of PSL (prednisolone) (or received corresponding steroid treatment) as a first-line treatment, and who falls under any of the following (1) through (4):

(1) A patient who needs additional treatment because of exacerbation even after 1 mg/kg or more of PSL has been administered for 2 weeks;
(2) A patient who has not been ameliorated after 4 weeks of treatment, including administration of 0.5 mg/kg or more of PSL for 2 weeks, and needs additional treatment.
(3) A patient whose GVHD symptom has relapsed or worsened within a period in which the dose of the steroid was reduced
(4) A steroid treatment-dependent patient for whom a dose reduction from 10 mg/day (or corresponding steroid treatment) is difficult

<Test design>

Tamibarotene was administered to 18 subjects. As the administration method, 2 mg tablets of tamibarotene were administered orally for 24 weeks. The administration was started with 2 doses (a 2 mg tablet, twice) after breakfast and

dinner, i.e., 4 mg (equivalent to 3 $mg/m^2$) per day. PSL or steroid administration was also continued daily.

<Administration method/ schedule>

2 mg tablets of tamibarotene were administered orally for 24 weeks. Administration was started at 4 mg (equivalent to 3 $mg/m^2$) per day, i.e., twice daily after breakfast and dinner (one 2 mg tablet in each of the morning and evening). If an adverse event was mild (grade 0 to 1), the dose could be increased to 8 mg per day (two 2 mg tablets in each of the morning and evening) after 4 weeks of continuation (in a case of 6 mg per day, two 2 mg tablets were to be taken after breakfast and one 2 mg tablet after dinner).

In a case of grade 2 for an unexpected adverse event (for which a causal relationship could not be ruled out), or grade 3 for an expected adverse event (for which a causal relationship could not be ruled out), the dose was reduced to 2 mg per day (one tablet once after breakfast), and administration was continued. Alternatively, after a 2-week drug holiday, administration was restarted at 2 mg per day and continued, after confirming that the adverse event had improved to a grade one level lower than that before the drug holiday. However, in regard to an abnormal liver function of grade 3, in a case of a patient who had grade 3 liver dysfunction due to chronic GVHD from before enrollment, administration could be continued without dose reduction or drug holiday, even if the abnormal liver function remained at grade 3. In a case where drug holidays and restarts were repeated with administration at 2 mg per day due to an adverse event, administration could be continued by reducing the dose to 2 mg on alternate days.

Adverse events include any unfavourable or unintended sign (including an abnormal value in clinical test), symptom, or disease that occurs when tamibarotene is administered.

Evaluation of the severity of adverse events was performed by grading in accordance with the common terminology criteria for adverse events v4.0 (CTCAE (CTCAE v4.03/MedDRA v12.0 (Japanese version: MedDRA/J v16.0))).

<Test evaluation items>

(FFS)

The survival rate without treatment failure (death, relapse, implementation of additional treatment) at the end of tamibarotene administration (week 24), i.e., failure-free survival (FFS), was evaluated. In a case where additional treatment was implemented for chronic GVHD, such a case was considered treatment failure at that point in time. In regard to the additional treatment herein, a change in the steroid dose was not considered, but in a case where the dose was increased to an amount exceeding 1 mg/kg/day, it was considered that additional treatment had been implemented.

(Chronic GVHD therapeutic effect in each organ)

A chronic GVHD clinical trial effect in each organ was determined under criteria indicated in Table 1.

[Table 1]

| Organ | CR | PR | SD | PD |
|---|---|---|---|---|
| Skin | 0 | S:3 → E:1 or 2<br>S:2 → E:1<br>S:1 → CR only | Other than CR, PR, and PD | E-S≥1 |
| Eyeball | 0 | S:3 → E:1 or 2<br>S:2 → E:1<br>S:1 → CR only | | E-S≥1 |
| Oral Cavity | 0 | S:≥8 → E/S≤0.5 and E>0<br>S:4 to 7 → S-E≥4 and E>0<br>S:≤3 → CR only | | E-S≥3 |
| Gastrointestinal Tract | 0 | S:3 → E:1 or 2<br>S:2 → E:1<br>S:1 → CR only | | E-S≥1 |

(continued)

| Organ | CR | PR | SD | PD |
|---|---|---|---|---|
| Liver Function | ≤ULN | S:≥(3 times of ULN) → E/S≤0.5 and E>0<br>S:<(3 times of ULN) → CR only | | S:≥(3 times of ULN) → E-S≥(3 times of ULN)<br>S:<(3 times of ULN) → E-S≥(2 times of ULN) |
| Lung | 2 | S:8 to 9 → E:3 or 4<br>S:6 to 7 → E:3<br>S:≤5 → CR only | | E-S≥3 |

In Table 1, "CR" stands for complete response, "PR" stands for partial response, "SD" stands for stable disease, and "PD" stands for progressive disease. "S" stands for a start time point, "E" stands for an end time point, and "ULN" stands for an upper limit of normal. For example, "S:3" indicates that the score at the start time point was 3. "CR only" indicates that there was no PR.

For the skin, eyeball, and gastrointestinal tract, the NIH organ-specific scores described in Biol. Blood Marrow Transplant, 2005 11(12), 945-56 were used. For the skin, a therapeutic effect was further evaluated using a total skin score. In addition, the 15-point Schubert scale in Table 2 was used for the oral cavity. For the liver function, clinical test values of T-Bil, ALP, and ALT were used, and for the lungs, the 12-point lung function score (LFS) was used.

[Table 2]

| Change in Oral Mucosa | No Chronic GVHD | | Mild Chronic GVHD | | Moderate Chronic GVHD | | Severe Chronic GVHD | |
|---|---|---|---|---|---|---|---|---|
| Erythema | None | 0 | Mild or Moderate (<25%) | 1 | Moderate or Severe | 2 | Severe (≥25%) | 3 |
| Lichen | None | 0 | Hyperkeratosis (<25%) | 1 | Hyperkeratosis (25 to 50%) | 2 | Hyperkeratosis (>50%) | 3 |
| Ulcer | None | 0 | - | - | ≤20% | 3 | >20% | 6 |
| Mucocele (evaluation of lower lip and soft palate only) | None | 0 | 1 to 5 | 1 | 6 to 10 | 2 | ≥11 | 3 |

For the total skin score, the body surface was subdivided into 10 areas (head and neck, chest, abdomen and genitals, back and buttocks, right arm, right hand, left arm, left hand, right lower limb, and left lower limb), and the chronic skin GVHD grade in each area was classified into the following 0 through 4. The proportion of an area occupied by each grade with respect to the entire area was also obtained.

- Grade 0: Normal skin
- Grade 1: Skin discoloration, depigmentation or hyperpigmentation, or hair loss
- Grade 2: lichenoid plaque, skin thickening, or mobile sclerosis
- Grade 3: Skin effect with reduced mobility, and the skin can be pinched
- Grade 4: Hard and contracted skin with no mobility, and the skin cannot be pinched

Furthermore, in a case where there was a lesion of grade 3 or 4, a range of an erythematous area therein was classified into 0%, 25%, 50%, 75%, or 100%.

(Chronic GVHD therapeutic effect)

Based on the criteria for the therapeutic effect on chronic GVHD for each organ shown in Table 1, the clinical trial effect on chronic GVHD at week 24 after the start of tamibarotene administration was determined using the determination criteria shown in Table 3.

[Table 3]

| Evaluation Item | Criteria |
|---|---|
| CR (Complete Response) | Evaluations of all chronic GVHD organs are CR, and no PD exists in any organ |
| PR (Partial Response) | Evaluation of at least one chronic GVHD organ is PR, and no PD exists in any organ |
| SD (Stable Disease) | Other than CR, PR, and PD |
| PD (Progressive Disease) | Evaluation of at least one chronic GVHD organ is PD, or onset of chronic GVHD in a new organ |
| UE (Unevaluable) | Organ damage cannot be definitively attributed to chronic GVHD, and other factors are highly involved; death or inability to observe early after start of treatment; data loss or loss to follow-up, etc. |

(Response rate)

The response rate was obtained using the following formula, based on the criteria shown in Table 3.

Response rate = [number of patients determined as complete response (CR) + number of patients determined as partial response (PR)]/number of patients who received treatment

In a case where leukemia or the like, which is "a primary disease for which hematopoietic stem cell transplantation is indicated" relapsed, such a case was considered treatment failure in the evaluation of FFS. However, in a case where no additional treatment such as re-transplantation or anticancer drug therapy was performed even after the relapse, and chronic GVHD was controlled, and it was determined that continuation of tamibarotene was suitable, administration was continued without discontinuation, and the response rate was determined.

(Secondary evaluation items)

The FFS and response rate at week 12 from the start of tamibarotene administration, as well as the FFS at week 24 from the start of tamibarotene administration, and the proportion of subjects for whom the dose of PSL could be reduced by 50% or more were also evaluated.

(Significance level of efficacy analysis)

Based on a model using Simon's two-stage design, the test was determined to be effective if there were 7 or more effective cases among all 18 subjects.

<Results>

Nine of the 18 subjects completed the clinical trial period (24 weeks). At weeks 12 and 24 of administration of tamibarotene, there were 5 subjects for whom the overall judgment was PR. At weeks 12 and 24 of administration of tamibarotene, there were 7 subjects for whom the overall judgment was SD. The failure-free survival (FFS) and the response rate at week 24 of administration of tamibarotene were each 28% (5/18).

In 3 subjects, the dose of PSL could be reduced by 50% or more at week 24 of administration of tamibarotene, as compared with that at the start of tamibarotene administration. Furthermore, in 7 subjects, the dose of PSL could be reduced at week 24 of administration of tamibarotene, as compared with that at the start of treatment.

(Overall response rate)

The overall response rate in the entire administered population at the time of the final analysis was 33.3% (6/18 o cases, 95% confidence interval: 13.3% to 59.0%).

(Overall response rate based on NIH Consensus Development Project Criteria (2014))

In the above test, the response was evaluated based on the NIH Consensus Development Project Criteria (2005). Therefore, a response evaluation based on the NIH Consensus Development Project Criteria (2014), which was

revised after the start of the test, was carried out as sensitivity analysis. The overall response rate based on the NIH Consensus Development Project Criteria (2014) in the entire administered population at the time of tumor evaluation was 38.9% (7/18 cases, 95% confidence interval: 17.3% to 64.3%), which was almost the same as the overall response rate based on the NIH Consensus Development Project Criteria (2005).

One of the 5 subjects (out of 11) whose overall judgment was PR was able to reduce the dose of PSL at 2 years after the end of treatment without exacerbation of chronic GVHD. Another subject was able to discontinue the administration of PSL and reduce the dose of FK506 at 2 years after the end of treatment without exacerbation of chronic GVHD.

(Chronic GVHD therapeutic effect in each organ)

<<Skin>>

At week 24 of administration of tamibarotene, the therapeutic effect was checked in 16 subjects. One subject out of the 16 subjects was evaluated as CR, and three subjects out of the 16 subjects were evaluated as PR. The NIH response rate was 25%. In evaluation based on the total skin score, the score was improved in 8 subjects out of the 16 subjects. In particular, the tamibarotene administration resulted in notable amelioration of edema, skin sclerosis, pigmentation, or erythema in several subjects.

<<Oral cavity>>

At week 24 of administration of tamibarotene, the therapeutic effect was checked in 16 subjects. Two subjects out of the 16 subjects were evaluated as CR, and two subjects out of the 16 subjects were evaluated as PR. The NIH response rate was 25%. In evaluation based on the 15-point Schubert scale, the score was improved in 10 subjects out of the 16 subjects. In particular, healing of ulcer and notable amelioration of erythema were seen in several subjects.

<<Eyeball>>

At week 24 of administration of tamibarotene, the therapeutic effect was checked in 14 subjects. No subject was evaluated as CR, and two subjects out of the 14 subjects were evaluated as PR. The NIH response rate was 14%. In evaluation based on the Schirmer's test, improvement was seen in 5 subjects out of the 18 subjects in the right eye and in 7 subjects out of the 18 subjects in the left eye.

<<Other organs>>

The NIH response rate was 20% for the gastrointestinal tract, 17% for the genitals, and 23% for the liver. The number of evaluable subjects was small for the gastrointestinal tract (n=5) and the genitals (n=6). The response rate for the liver was 23%, but it was difficult to consider the influence by other diseases. The response rate for the lung function score was unchanged. The NIH response rate for the joints and fascia was 11%, while a notable improvement in the range of joint motion was seen in subjects in whom a therapeutic effect was confirmed.

<Summary of Example 1>

From the above result, the effect of tamibarotene on chronic GVHD was confirmed. In particular, remarkable amelioration was seen in skin and oral cavity lesions in many subjects, and amelioration was also seen in eyeball lesions in subjects.

Moreover, many of the subjects were able to reduce the steroid dose, and it was found that by use of tamibarotene in combination with a steroid, the steroid dose can be reduced as compared with that in administration of a steroid alone.

[Example 2: Chronic GVHD therapeutic effect in each organ]

The chronic GVHD therapeutic effect in each organ was investigated in specific cases of Example 1.

<Case 1: Improvement in range of motion of knee joint>

The dose shown below was administered to the patient.

At start of administration (tamibarotene/steroid/tacrolimus): 4 mg/15 mg/4 mg
At end of administration (tamibarotene/steroid/tacrolimus): 2 mg (on alternate days)/3 mg/3 mg

Steroid: prednisolone
Administration period: 200 days (total dose of tamibarotene: 356 mg)

Fig. 1 is a diagram illustrating comparisons of knee joint extension and knee joint flexion between start of administration and end of administration.
As shown in Fig. 1, tamibarotene administration resulted in notable improvement in knee joint extension and notable improvement in knee joint flexion.

<Case 2: Improvement of cutis symptom>

The dose shown below was administered to the patient.

At start of administration (tamibarotene/steroid/tacrolimus): 4 mg/11 mg/0.2 mg
At end of administration (tamibarotene/steroid/tacrolimus): 0 mg/9 mg/0.6 mg
Steroid: predonine
Administration period: 137 days (total dose of tamibarotene: 386 mg)

Fig. 2 is a diagram illustrating comparisons of a cutis symptom on a lower part of a back and a cutis symptom on a knee between start of administration and end of administration.
As shown in Fig. 2, tamibarotene administration resulted in amelioration of pigmentation and skin sclerosis in the lower part of the lower back and notable amelioration of pigmentation and skin sclerosis in the knee.

<Case 3: Amelioration in oral cavity symptom>

The dose shown below was administered to the patient.

At start of administration (tamibarotene/steroid/tacrolimus): 4 mg/10 mg/0.6 mg
At end of administration (tamibarotene/steroid/tacrolimus): 4 mg/10 mg/0.6 mg
Steroid: prednisolone
Administration period: 83 days (total dose of tamibarotene: 332 mg)

Fig. 3 is a diagram illustrating a comparison of an oral cavity symptom between start of administration and end of administration.
As shown in Fig. 3, healing of ulcer and notable amelioration of erythema were seen in the oral cavity.

<Other functions>

The response rate for each organ was calculated from all cases in Example 1.
Fig. 4 indicates the response rate for each organ.
As shown in the graph in Fig. 4, the effect was seen in chronic GVHD of the eyeball and liver in addition to the skin and the oral cavity, or chronic GVHD maintained without exacerbation.

<Summary of Example 2>

As a first-line treatment for chronic GVHD, steroid is administered to suppress the inflammatory response. However, a second-line treatment for a case where a side effect due to steroid administration has occurred or a disease condition is exacerbated has not been established. Although there is an individual difference, the disease condition progresses in the long term, while the disorder (inflammatory response) of each organ repeats exacerbation and remission.
No amelioration was seen in any of the patients in Examples before administration of tamibarotene, even though various drugs had been administered with respect to a lesion in each organ. However, amelioration in the symptom was seen in all the patients after administration of tamibarotene.
For chronic GVHD, it is assumed that, in a stage in which immunity is reconstituted in a body of a recipient by donor cells, autoantigen reactive T cells appear, and a clinical picture which is similar to an autoimmune disease is shown. Th 17 cells which are a subset of helper T cells are known as a factor involved in autoimmune diseases. It is considered that tamibarotene suppresses autoimmune diseases by inhibiting the differentiation of T cells into Th 17 cells. In a case where such an action is observed, it is considered that amelioration of chronic GVHD by a new mechanism, in addition to steroids and immune-suppressing drugs currently administered for alleviation of symptoms of chronic GVHD, will lead to a

reduction in doses of these drugs.

[Example 3: Investigation of plasma concentration of active ingredient]

To a total of 11 patients, a single dose of 2.0 mg, 4.0 mg, or 6.0 mg of tamibarotene was administered.
Moreover, to a total of 6 patients, repeated doses of 8.0 mg or 10.0 mg of tamibarotene were administered.
For the 2.0 mg dose, one tablet (2 mg of tamibarotene) was administered after breakfast using a tablet of 2 mg tamibarotene; for the 4.0 mg dose, one tablet (2 mg of tamibarotene) was administered after breakfast and one tablet (2 mg of tamibarotene) was administered after dinner; and for the 6.0 mg dose, two tablets (4 mg of tamibarotene) were administered after breakfast and one tablet (2 mg of tamibarotene) was administered after dinner.
For the 8.0 mg dose, two tablets (4 mg of tamibarotene) after breakfast and two tablets (4 mg of tamibarotene) after dinner were administered repeatedly for 35 to 46 days, using a tablet of 2 mg tamibarotene. For the 10.0 mg dose, three tablets (6 mg of tamibarotene) after breakfast and two tablets (4 mg of tamibarotene) after dinner were administered repeatedly for 34 to 59 days, using a tablet of 2 mg tamibarotene.
After administration of tamibarotene to each patient, blood was collected on a specific day at 0, 2, 4, 8, 10, and 24 hours, and the concentration of tamibarotene in human plasma was measured by a high-performance liquid chromatography (HPLC) method.

<Results of Example 3>

Plasma concentration of the active ingredient was maintained at 1 ng/mL to 200 ng/mL in all the patients during the dosing period.

<Measurement result of lowest plasma concentration of active ingredient>

- Plasma concentration after 24 hours from single dose of 6.0 mg: 1.08 ng/mL
- Plasma concentration after 24 hours from single dose of 6.0 mg: 1.14 ng/mL
- Plasma concentration after 24 hours from a single dose of 4.0 mg: 2.09 ng/mL
- Plasma concentration after 24 hours from single dose of 6.0 mg: 2.50 ng/mL
- Plasma concentration after 10 hours from a single dose of 2.0 mg: 2.23 ng/mL
- Plasma concentration after 10 hours from a single dose of 2.0 mg: 3.05 ng/mL
- Plasma concentration after 8 hours from a single dose of 2.0 mg: 3.26 ng/mL
- Plasma concentration after 24 hours from a single dose of 4.0 mg: 4.95 ng/mL
- Plasma concentration after 10 hours from a single dose of 2.0 mg: 4.66 ng/mL
- Plasma concentration after 8 hours from a single dose of 2.0 mg: 5.04 ng/mL
- Plasma concentration after 24 hours from repeated doses (46 days) of 8.0 mg: 2.92 ng/mL
- Plasma concentration after 4 hours from a single dose of 2.0 mg: 8.98 ng/mL
- Plasma concentration after 10 hours from a single dose of 4.0 mg: 9.56 ng/mL
- Plasma concentration after 10 hours from a single dose of 4.0 mg: 8.03 ng/mL
- Plasma concentration after 10 hours from a single dose of 4.0 mg: 7.01 ng/mL
- Plasma concentration after 6 hours from a single dose of 6.0 mg: 7.42 ng/mL
- Plasma concentration after 10 hours from a single dose of 6.0 mg: 7.72 ng/mL

<Measurement result of highest plasma concentration of active ingredient>

- Plasma concentration after 2 hours from repeated doses (58 days) of 10.0 mg: 199.2 ng/mL
- Plasma concentration after 4 hours from repeated doses (36 days) of 10.0 mg: 161.1 ng/mL
- Plasma concentration after 2 hours from repeated doses (59 days) of 10.0 mg: 118.8 ng/mL
- Plasma concentration after hours from repeated doses (46 days) of 8.0 mg: 100.09 ng/mL
- Plasma concentration after 8 hours from repeated doses (36 days) of 10.0 mg: 111.99 ng/mL
- Plasma concentration after 4 hours from repeated doses (47 days) of 6.0 mg: 92.53 ng/mL
- Plasma concentration after 4 hours from repeated doses (58 days) of 10.0 mg: 88.74 ng/mL
- Plasma concentration after 4 hours from repeated doses (59 days) of 10.0 mg: 82.06 ng/mL
- Plasma concentration after 2 hours from repeated doses (34 days) of 10.0 mg: 88.02 ng/mL

- Plasma concentration after 4 hours from repeated doses (34 days) of 10.0 mg: 85.64 ng/mL
- Plasma concentration after 4 hours from repeated doses (35 days) of 8.0 mg: 84.6 ng/mL
- Plasma concentration after 4 hours from repeated doses (46 days) of 8.0 mg: 85.21 ng/mL
- Plasma concentration after 4 hours from a single dose of 6.0 mg: 80.21 ng/mL

<Other measurement results>

- After 2, 4, 8, 10 hours from a single dose of 2.0 mg, after 2, 4, 8 hours from a single dose of 4.0 mg, after 2, 4, 8, 10 hours from a single dose of 6.0 mg, after 0, 2, 4, 8, 10 hours from repeated doses of 8.0 mg, and after 0, 2, 4, 8, 10 hours from repeated doses of 10.0 mg, the plasma concentration fell within a range between 10 ng/mL and 80 ng/mL.

Industrial Applicability

The present invention can be utilized, for example, in treatment and research of graft versus host disease.

## Claims

1. A therapeutic agent for human graft versus host disease, said therapeutic agent comprising tamibarotene or a pharmaceutically acceptable salt thereof as an active ingredient.

2. The therapeutic agent as set forth in claim 1 which is used in combination with at least one selected from a steroid drug and an immune-suppressing drug.

3. The therapeutic agent as set forth in claim 1 or 2, wherein:
the human graft versus host disease is chronic graft versus host disease.

4. The therapeutic agent as set forth in claim 1, which suppresses progression of or ameliorates a lesion derived from the human graft versus host disease in at least one selected from the group consisting of skin, an oral cavity, eyeballs, fasciae, joints, and lungs.

5. The therapeutic agent as set forth in claim 1, wherein:
a plasma concentration of the active ingredient is maintained at 1 ng/mL to 200 ng/mL during a dosing period in a subject receiving a treatment.

6. The therapeutic agent as set forth in claim 1, which is administered to a subject with a drug holiday period or a dose reduction period.

7. The therapeutic agent as set forth in claim 6, wherein:
during the dose reduction period, a plasma concentration of the active ingredient is maintained at 1 ng/mL or more in the subject to which said therapeutic agent has been administered.

8. The therapeutic agent as set forth in claim 2, wherein:

   the steroid drug is selected from the group consisting of prednisolone, methylprednisolone, clobetasol propionate, diflorasone acetate, betamethasone dipropionate, diflubrednate, diflucortolone valerate, fluocinonide, amcinonide, hydrocortisone butyrate propionate, betamethasone acetate propionate, mometasone furoate, betamethasone valerate-gentamicin sulfate, betamethasone valerate, dexamethasone propionate, beclomethasone propionate, dexamethasone valerate, fluocinolone acetonide, debrodone propionate, triamcinolone acetonide, hydrocortisone butyrate, clobetasone butyrate, alclometasone propionate, prednisolone valerate acetate, and dexamethasone; and
   the immune-suppressing drug is selected from the group consisting of cyclosporine, tacrolimus, mycophenolate mofetil, sirolimus, pentostatin, and methotrexate.

9. The therapeutic agent as set forth in claim 2, wherein:
a daily dose of the steroid drug used in combination with said therapeutic agent is reduced as compared with a daily

dose of the steroid drug needed when used alone for treatment.

**10.** The therapeutic agent as set forth in claim 2, wherein:
a daily dose of the steroid drug used in combination with said therapeutic agent is reduced by 10% or more as compared with a daily dose of the steroid drug needed when used alone for treatment.

**11.** The therapeutic agent as set forth in claim 2, wherein:
at or after 24 weeks from start of administration of said therapeutic agent, a daily dose of the steroid drug used in combination with said therapeutic agent is reduced by 10% or more as compared with a daily dose of the steroid drug used in combination with said therapeutic agent at the start of administration of said therapeutic agent.

**12.** The therapeutic agent as set forth in claim 2, wherein:
a daily dose of the steroid drug used in combination with said therapeutic agent is reduced stepwise.

**13.** The therapeutic agent as set forth in claim 1, which is administered to any one of:

1) a subject for whom a daily dose of the steroid drug is difficult to reduce from 10 mg/kg in terms of prednisolone;
2) a subject for whom exacerbation is seen even when a daily dose of the steroid drug is 1 mg/kg in terms of prednisolone and to whom the steroid drug has been administered daily for 2 weeks; and
3) a subject whose symptom is not ameliorated even when a daily dose of the steroid drug is 0.5 mg/kg in terms of prednisolone and the steroid drug has been administered daily for 4 to 8 weeks.

**14.** A combination of a therapeutic agent for human graft versus host disease, said combination comprising:

at least one selected from a steroid drug and an immune-suppressing drug; and
a therapeutic agent recited in claim 2.

FIG. 1

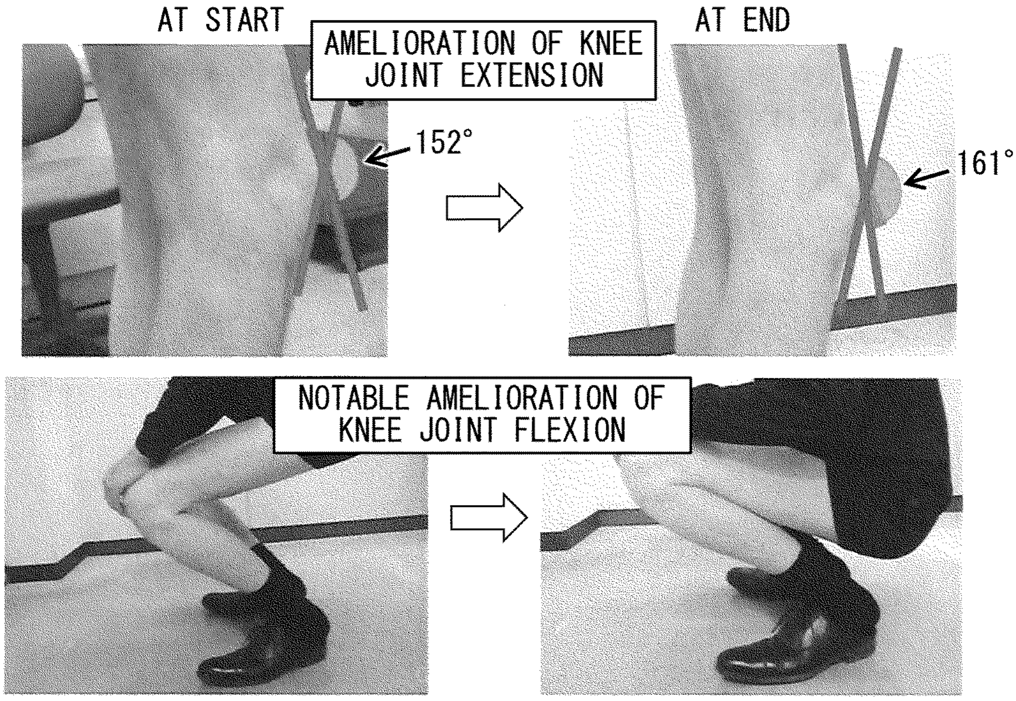
AT START
AT END
AMELIORATION OF KNEE JOINT EXTENSION
152°
161°
NOTABLE AMELIORATION OF KNEE JOINT FLEXION

FIG. 2

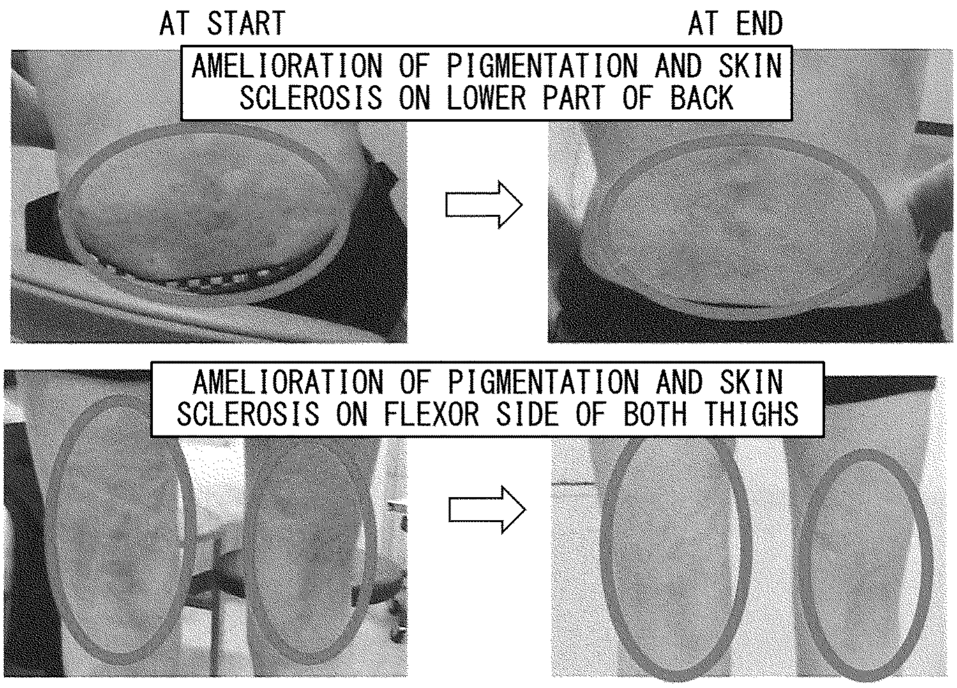
AT START
AT END
AMELIORATION OF PIGMENTATION AND SKIN SCLEROSIS ON LOWER PART OF BACK
AMELIORATION OF PIGMENTATION AND SKIN SCLEROSIS ON FLEXOR SIDE OF BOTH THIGHS

FIG. 3

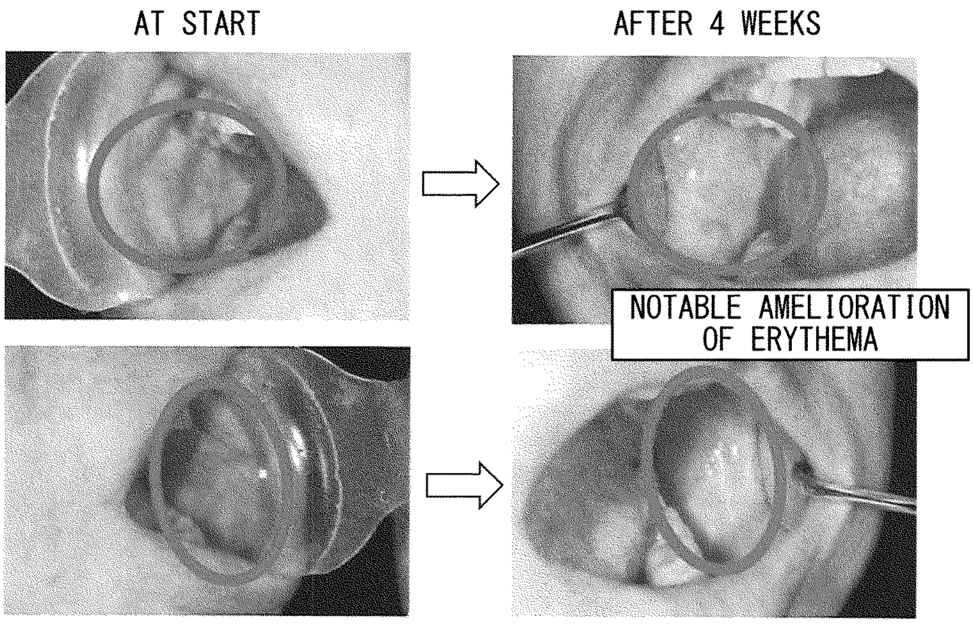
AT START
AFTER 4 WEEKS
NOTABLE AMELIORATION OF ERYTHEMA

FIG. 4

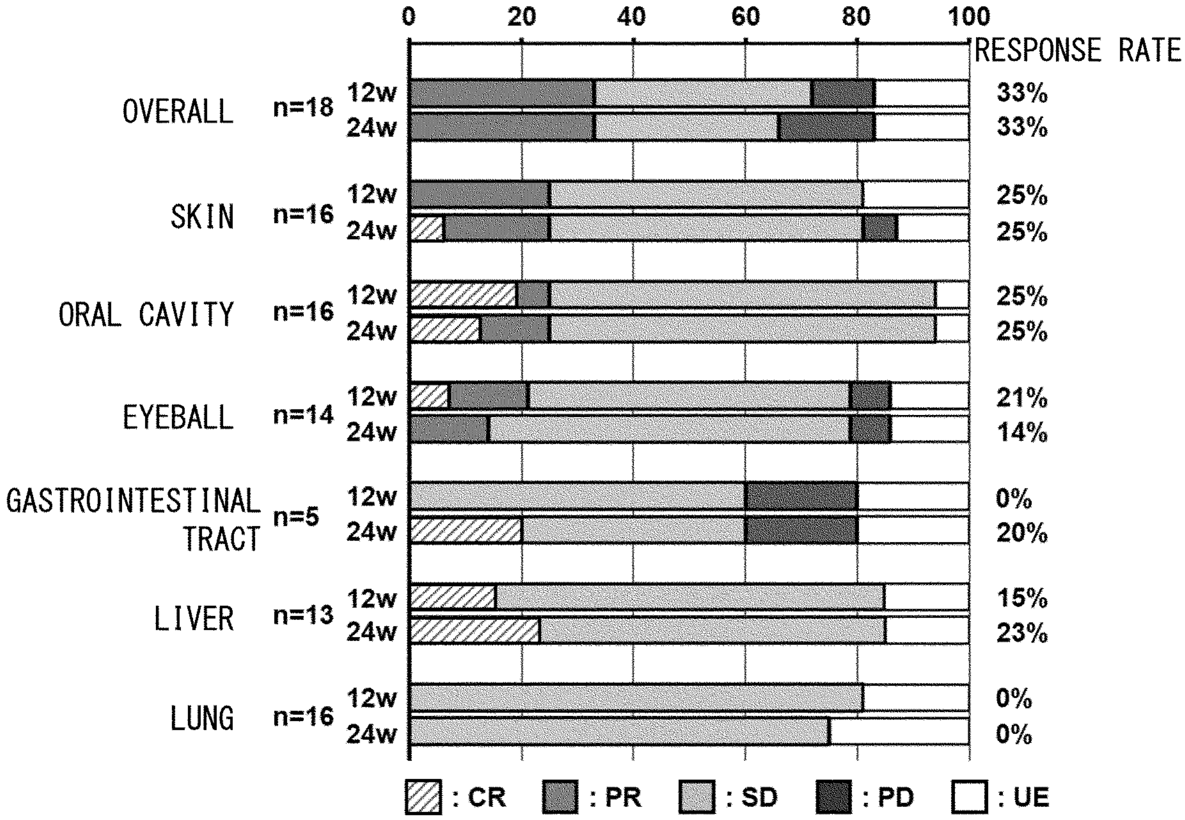
0
20
40
60
80
100
RESPONSE RATE
OVERALL n=18 12w 33%
24w 33%
SKIN n=16 12w 25%
24w 25%
ORAL CAVITY n=16 12w 25%
24w 25%
EYEBALL n=14 12w 21%
24w 14%
GASTROINTESTINAL TRACT n=5 12w 0%
24w 20%
LIVER n=13 12w 15%
24w 23%
LUNG n=16 12w 0%
24w 0%
: CR
: PR
: SD
: PD
: UE

**INTERNATIONAL SEARCH REPORT**

International application No. **PCT/JP2024/025206**

**A. CLASSIFICATION OF SUBJECT MATTER**

***A61K 31/192***(2006.01)i; ***A61K 31/57***(2006.01)i; ***A61K 31/58***(2006.01)i; ***A61K 31/436***(2006.01)i; ***A61K 31/519***(2006.01)i; ***A61K 31/573***(2006.01)i; ***A61K 31/5377***(2006.01)i; ***A61K 31/7056***(2006.01)i; ***A61K 38/13***(2006.01)i; ***A61K 45/00***(2006.01)i; ***A61P 37/06***(2006.01)i; ***A61P 43/00***(2006.01)i

FI: A61K31/192; A61P37/06; A61K31/573; A61P43/00 121; A61K45/00; A61K31/57; A61K31/58; A61K38/13; A61K31/7056; A61K31/519; A61K31/5377; A61K31/436

According to International Patent Classification (IPC) or to both national classification and IPC

**B. FIELDS SEARCHED**

Minimum documentation searched (classification system followed by classification symbols)

A61K31/192; A61K31/57; A61K31/58; A61K31/436; A61K31/519; A61K31/573; A61K31/5377; A61K31/7056; A61K38/13; A61K45/00; A61P37/06; A61P43/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | WATANABE, Satoshi et al. Tamibarotene for the Treatment of Bronchiolitis Obliterans Associated With Chronic Graft-vs-Host Disease. CHEST [online], 2019, 155(1), pp. e1-e4, Internet, URL<DOI: https://doi.org/10.1016/j.chest.2018.08.1052> title, abstract, p. e1, left column, last line, fig. 1A-D, 2, p. e2, lower right, line 2 from the bottom to p. e3, left column, line 10 | 1-8, 14 |
| Y | | 9-13 |
| X | NISHIMORI, Hisakazu et al. Synthetic retinoid Am80 ameliorates chronic graft-versus-host disease by down-regulating Th1 and Th17. Blood. 2012, 119(1), pp. 285-295 title, abstract, each figure, each table, final sentence of the main text, etc. | 1, 3-7 |
| Y | | 2, 8-14 |

☑ Further documents are listed in the continuation of Box C. ☐ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **29 August 2024** | **10 September 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

International application No.
**PCT/JP2024/025206**

**C. DOCUMENTS CONSIDERED TO BE RELEVANT**

| Category* | Citation of document, with indication, where appropriate, of the relevant passages | Relevant to claim No. |
|---|---|---|
| X | 西森 久和 ほか, 合成レチノイドAm80はTh1とTh17を抑制することにより慢性移植片対宿主病を改善する, 岡山医学会雑誌, 2012, vol. 124, pp. 197-201, (NISHIMORI, Hisakazu et al. Synthetic retinoid Am80 ameliorates chronic graft-versus-host disease by downregulating Th1 and Th17. Journal of Okayama Medical Association.) title, section "Introduction", p. 200, right column, section "Chronic GVHD prevention and treatment effect by Am80", each figure, section "Conclusion" | 1, 3-7 |
| Y | | 2, 8-14 |
| X | 西森 久和 ほか, 慢性移植片対宿主病に対するタミバロテン（AM80G）の医師主導臨床第II相試験, 岡山医学会雑誌, 2015, vol. 127, pp. 133-137, (NISHIMORI, Hisakazu et al. An investigator initiated phase II clinical trial of tamibarotene (AM80G) for chronic graft-versus-host disease. Journal of Okayama Medical Association.) title, section "Introduction", p. 134, section "Mechanism of Tamibarotene on chronic GVHD", pp. 135-136, left column, sections "Trial design", "Conclusion", each figure, tech table, etc. | 1-14 |
| Y | | 2, 8-14 |
| X | MAEDA, Yoshinobu et al. An Open-labeled, Multicenter Phase II Study of Tamibarotene in Patients with Steroid-refractory Chronic Graft-versus-Host Disease. Acta Med. Okayama. 2016, vol. 70, no. 5, pp. 409-412 title, abstract, p. 410, left column, lines 13-22, p. 411, right column, line 14-20, etc. | 1-14 |
| Y | | 2, 8-14 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 3001632 B
- JP 61076440 A
- WO 2002018322 A


### Non-patent literature cited in the description


- Hematopoietic Cell Transplantation Guideline GVHD. The Japan Society for Hematopoietic Cell Transplantation, November 2022
- *Biol. Blood Marrow Transplant*, 2005, vol. 11 (12), 945-56